(19) Europäisches Patentamt / European Patent Office / Office européen des brevets

(11) **EP 1 771 109 B1**

(12) **EUROPEAN PATENT SPECIFICATION**

(45) Date of publication and mention
of the grant of the patent:
**16.04.2014 Bulletin 2014/16**

(21) Application number: **05772104.5**

(22) Date of filing: **07.07.2005**

(51) Int Cl.:
*A61B 5/1455* (2006.01)　　　*A61B 5/00* (2006.01)

(86) International application number:
**PCT/US2005/024088**

(87) International publication number:
**WO 2006/017117 (16.02.2006 Gazette 2006/07)**

(54) **CYANOTIC INFANT SENSOR**

ZYANOSE-SENSOR FÜR SÄUGLINGE

CAPTEUR DE NOURRISSON CYANOSÉ

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **09.07.2004 US 586281 P**

(43) Date of publication of application:
**11.04.2007 Bulletin 2007/15**

(73) Proprietor: **Masimo Corporation
Irvine, CA 92618 (US)**

(72) Inventor: **AL-ALI, Ammar
Tustin, California 92782 (US)**

(74) Representative: **Vossius & Partner
Siebertstrasse 4
81675 München (DE)**

(56) References cited:
**WO-A-02/35999　　　WO-A-97/03603
WO-A-02/089664　　　US-A- 5 437 275
US-A1- 2002 173 708　　US-B1- 6 256 523**

**Description**

BACKGROUND OF THE INVENTION

Cyanosis is a congenital condition in which blood pumped to the body contains less than normal amounts of oxygen, resulting in a blue discoloration of the skin. The most common cyanotic condition is tetralogy of Fallot, which is characterized by an abnormal opening, or ventricular septal defect, that allows blood to pass from the right ventricle to the left ventricle without going through the lungs; a narrowing, or stenosis, proximate the pulmonary valve, which partially blocks the flow of blood from the right side of the heart to the lungs; a right ventricle that is abnormally muscular; and an aorta that lies directly over the ventricular septal defect. Another cyanotic condition is tricuspid atresia, characterized by a lack of a tricuspid valve and resulting in a lack of blood flow from the right atrium to the right ventricle. Yet another cyanotic condition is transposition of the great arteries, i.e. the aorta originates from the right ventricle, and the pulmonary artery originates from the left ventricle. Hence, most of the blood returning to the heart from the body is pumped back out without first going to the lungs, and most of the blood returning from the lungs goes back to the lungs.

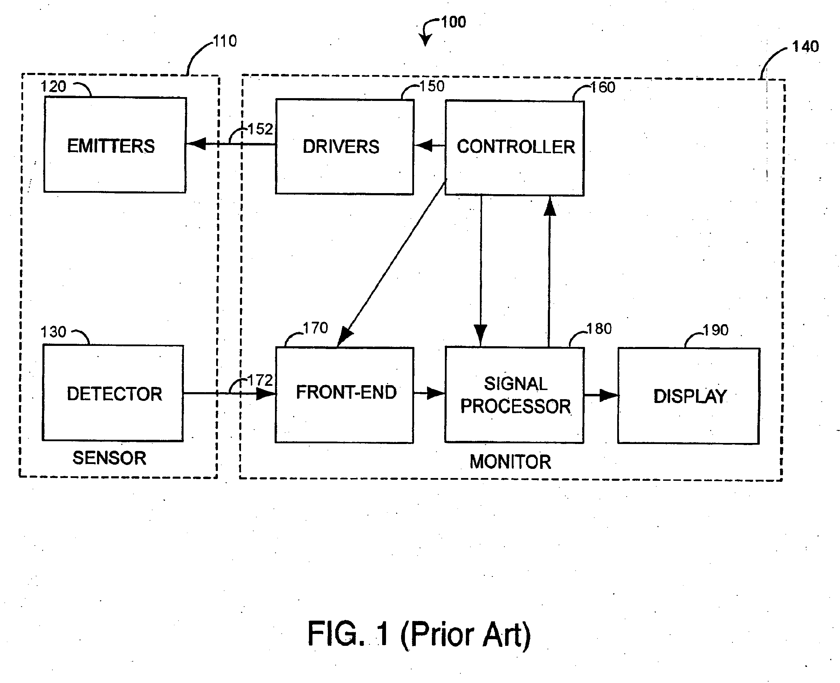

Pulse oximetry is a useful tool for diagnosing and evaluating cyanotic conditions. A pulse oximeter performs a spectral analysis of the pulsatile component of arterial blood so as to measure oxygen saturation, the relative concentration of oxygenated hemoglobin, along with pulse rate. FIG. **1** illustrates a pulse oximetry system **100** having a sensor **110** and a monitor **140**. The sensor **110** has emitters **120** and a detector **130** and is attached to a patient at a selected fleshy tissue site, such as a thumb or toe. The emitters **120** project light through the blood vessels and capillaries of the tissue site. The detector **130** is positioned so as to detect the emitted light as it emerges from the tissue site. A pulse oximetry sensor is described in U.S. Patent 6,088,607 entitled "Low Noise Optical Probe," which is assigned to Masimo Corporation, Irvine, CA.

Also shown in FIG. **1**, the monitor **140** has drivers **150**, a controller **160**, a front-end **170**, a signal processor **180**, a display **190**. The drivers **150** alternately activate the emitters **120** as determined by the controller **160**. The front-end **170** conditions and digitizes the resulting current generated by the detector **130**, which is proportional to the intensity of the detected light. The signal processor **180** inputs the conditioned detector signal and determines oxygen saturation, as described below, along with pulse rate. The display 190 provides a numerical readout of a patient's oxygen saturation and pulse rate. A pulse oximetry monitor is described in U.S. Patent 5,482,036 entitled "Signal Processing Apparatus and Method," which is assigned to Masimo Corporation, Irvine, CA. Other pulse oximetry sensors are described in US 6 256 523, WO 97/03603 and US 5 437 275.

SUMMARY OF THE INVENTION

The Beer-Lambert law provides a simple model that describes a tissue site response to pulse oximetry measurements. The Beer-Lambert law states that the concentration $c_i$ of an absorbent in solution can be determined by the intensity of light transmitted through the solution, knowing the mean pathlength, $mpl_\lambda$, the intensity of the incident light, $I_{0,\lambda}$, and the extinction coefficient, $\varepsilon_{i,\lambda}$, at a particular wavelength $\lambda$. In generalized form, the Beer-Lambert law is expressed as:

$$I_\lambda = I_{o,\lambda} e^{-mpl_\lambda \cdot \mu_{a,\lambda}} \qquad\qquad (1)$$

$$\mu_{a,\lambda} = \sum_{i=1}^{n} \varepsilon_{i,\lambda} \cdot c_i \qquad\qquad (2)$$

where is the bulk absorption coefficient and represents the probability of absorption per unit length. For conventional pulse oximetry, it is assumed that there are only two significant absorbers, oxygenated hemoglobin ($HbO_2$) and reduced hemoglobin (Hb). Thus, two discrete wavelengths are required to solve EQS. **1-2**, e.g. red (RD) and infrared (IR).

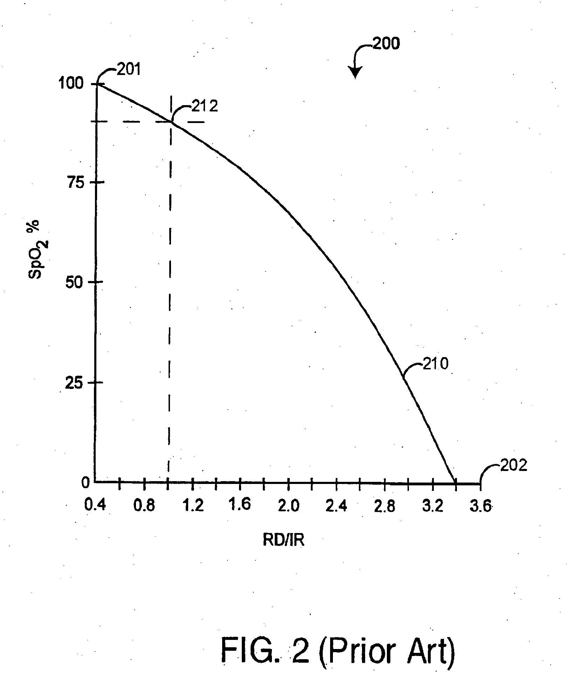

FIG. **2** shows a graph **200** depicting the relationship between RD/IR **202** and oxygen saturation ($SpO_2$ **201**, where RD/IR denotes the ratio of the DC normalized, AC detector responses to red and infrared wavelengths, as is well-known in the art and sometimes referred to as the "ratio-of-ratios." This relationship can be approximated from Beer-Lambert's Law, described above. However, it is most accurately determined by statistical regression of experimental measurements obtained from human volunteers and calibrated measurements of oxygen saturation. The result can be depicted as a curve **210**, with measured values of RD/IR shown on an x-axis **202** and corresponding saturation values shown on a y-axis **201**. In a pulse oximeter device, this empirical relationship can be stored in a read-only memory (ROM) for use as a look-up table so that $SpO_2$ can be directly read-out from an input RD/IR measurement. For example, an RD/IR value of 1.0 corresponding to a point **212** on the calibration curve **210** indicates a resulting $SpO_2$ value of approximately 85%.

Accurate and consistent pulse oximetry measurements on cyanotic infants have been difficult to obtain. An assumption inherent in the calibration curve **210** (FIG. **2**) is that the mean pathlength ratio for RD and IR is constant across the patient population. That is:

$$mpl_{RD}\Big/mpl_{IR} = C \qquad\qquad (3)$$

However, EQ. 3 may not be valid when cyanotic infants are included in that population. The reason may lie in what has been observed as abnormal tissue tone or lack of firmness associated with cyanotic defects, perhaps due to reduced tissue fiber. Such differences in tissue structure may alter the mean pathlength ratio as compared with normal infants. A cyanotic infant sensor addresses these problems by limiting variations in the RD over IR mean pathlength ratio and/or by providing a mean pathlength ratio measure so as to compensate for such variations. Alone or combined, these sensor apparatus and algorithms increase the accuracy and consistency of pulse oximetry measurements for cyanotic infants.

BRIEF DESCRIPTION OF THE DRAWINGS

FIG. **1** is a block diagram of a prior art pulse oximetry system;
FIG. **2** is an exemplar graph of a conventional calibration curve;
FIGS. **3A-B** are a perspective and an exploded perspective views, respectively, of a cyanotic infant sensor embodiment;
FIGS. **4-5** depict cross-sectional views of a tissue site and an attached pulse oximeter sensor, respectively;
FIG. **6** depicts a cross-sectional view of a tissue site and an attached cyanotic infant sensor;
FIGS. **7A-B** are plan and cross-sectional sensor head views of a conventional pulse oximeter sensor;
FIGS. **8-9** are plan and cross-sectional sensor head views of cyanotic infant sensor embodiments; and
FIG. **10** is an exemplar graph of a calibration surface incorporating a mean pathlength ratio measure.

DETAILED DESCRIPTION OF THE PREFERRED EMBODIMENTS

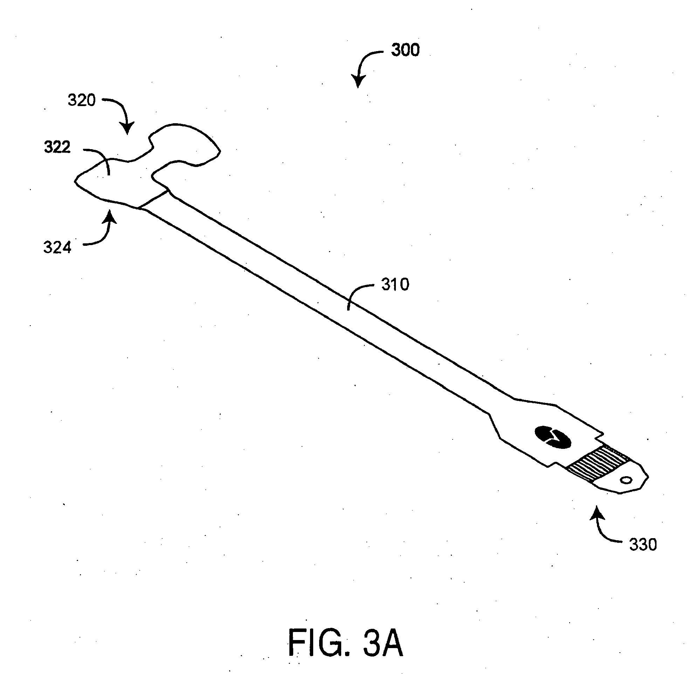

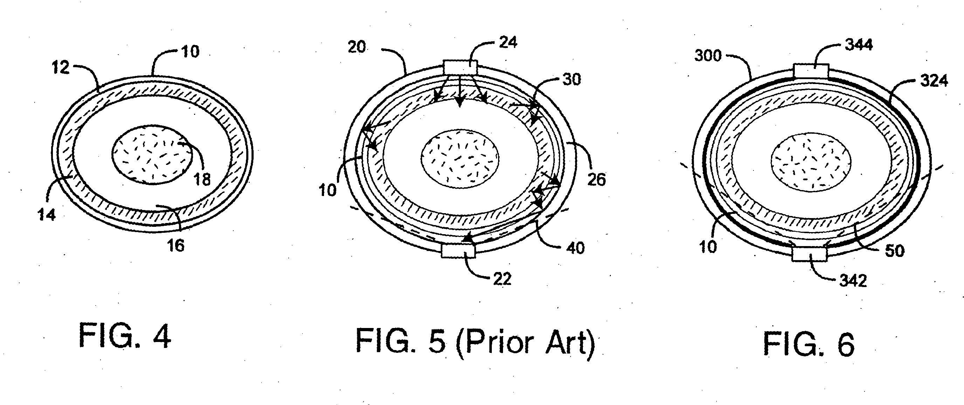

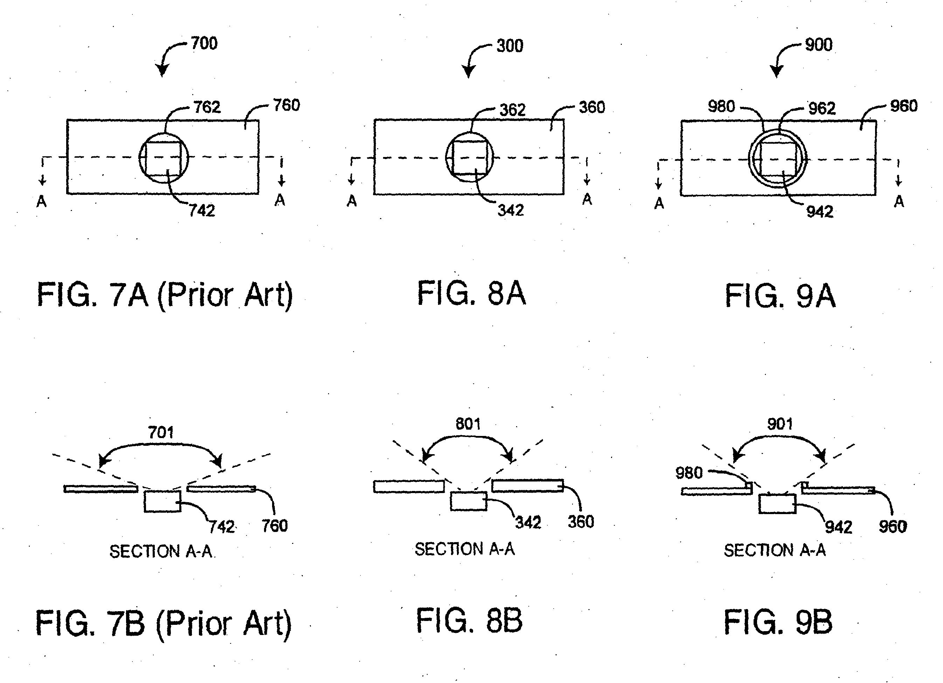

FIGS. **3A-B** illustrate one embodiment of a cyanotic infant sensor. The sensor has a light absorbing surface, as described with respect to FIGS. **4-6**, below. The sensor also has a detector window configured to limit the detector field of view (FOV), as described with respect to FIGS. **7-9**, below. Advantageously, these features limit mean pathlength ratio variations that are particularly manifest in cyanotic patients.

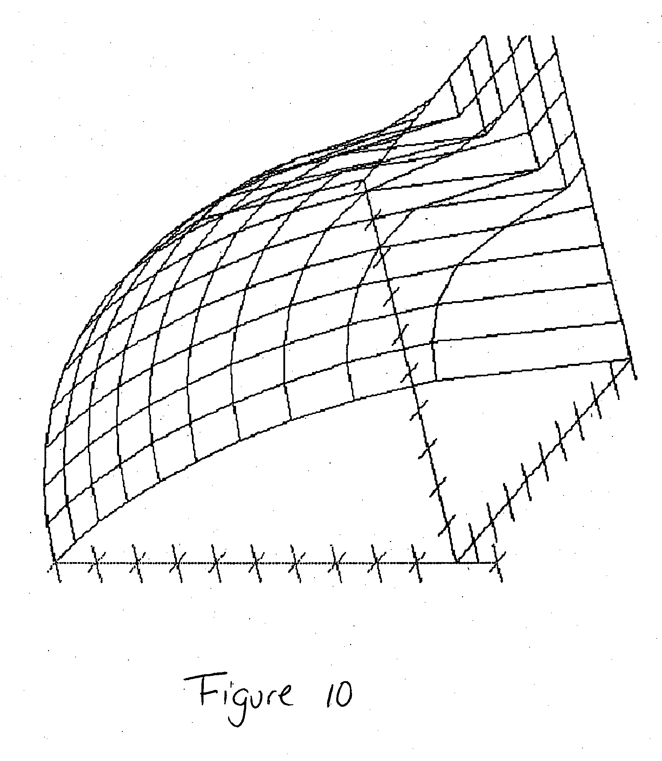

The sensor emitters and detector are also matched so as to limit variations in the detector red over IR DC response, i.e. $RD_{DC}/IR_{DC}$, that are not attributed to variations in the mean pathlength ratio (EQ. **3**). Such matching advantageously allows for measurement and calibration of the mean pathlength ratio, as described with respect to FIG. **10**, below. In one embodiment, cyanotic infant sensors 300 are constructed so that:

$$\lambda_{RD} \approx c_1 \,;\; \lambda_{IR} \approx c_2 \qquad\qquad (4)$$

$$I_{0,RD}\Big/I_{0,IR} \approx c_3\,;\; \text{for } i_{DC}(RD),\, i_{DC}(IR) \qquad\qquad (5)$$

$$RD_{DC}\,/\,IR_{DC} \approx c_4 \qquad\qquad (6)$$

That is, sensors 300 are constructed from red LEDs and IR LEDs that are each matched as to wavelength (EQ. 4). The LEDs are further matched as to red over IR intensity for given DC drive currents (EQ. 5). In addition, the sensors 300 are constructed from detectors that are matched as to red over IR DC response (EQ. 6).

As shown in FIG. 3A, the sensor 300 has a body 310 physically connecting and providing electrical communication between a sensor head 320 and a connector 330. The sensor head 320 houses the emitters and detector and attaches to a patient tissue site. The connector mates with a patient cable so as to electrically communicate with a monitor. In one embodiment, a sensor head surface 324 is constructed of light absorbing material.

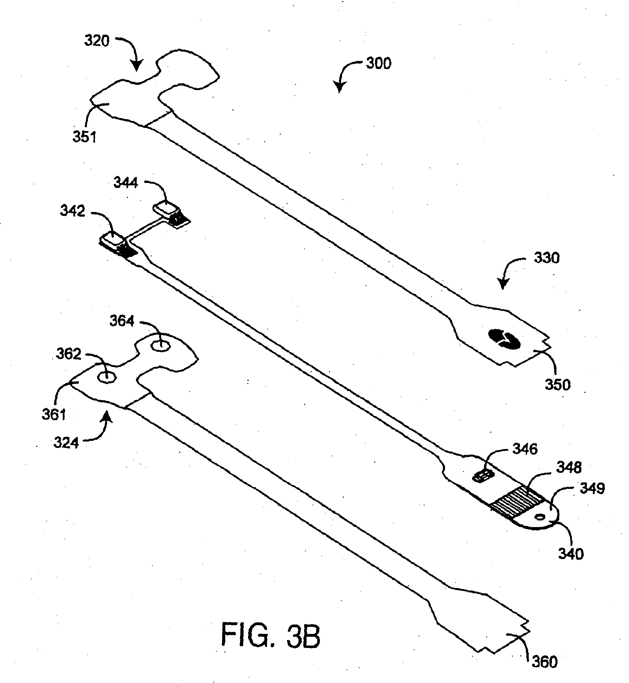

As shown in FIG. 3B, the sensor 300 has a face tape 330, a flex circuit 340 and a base tape 360, with the flex circuit 340 disposed between the face tape 330 and the base tape 360. The flex circuit 340 has a detector 342, an emitter 344 with at least two light emitting diodes (LEDs), an information element 346, and contacts 348 disposed on a connector tab 349. Neonatal sensors having a detector, LEDs, an information element, contacts and connector tab are described in U.S. Patent 6,256,523 entitled "Low-Noise Optical Probes," which is assigned to Masimo Corporation, Irvine, CA. In one embodiment, the face tape 350 and base tape 360 are constructed of Betham tape having attached polyethylene head tapes 351, 361. In a particular embodiment, the base head tape 361 is made of black polyethylene, and the face head tape 351 is made of white polyethylene. In one embodiment, a clear tape layer is disposed on the base head tape 361 tissue side over the detector window 362. The base head tape 361 has a detector window 362 and an emitter window 364 each allowing light to pass through the base head tape 361. In one embodiment, the base head tape 361 has a 4 mil thickness and the flex circuit has a 10 mil thickness. The combined 14 mil material thickness functions to limit the detector FOV, as described with respect to FIGS. 6 and 8, below.

FIGS. 4-6 illustrate some of the pathlength control aspects of a cyanotic infant sensor 300. FIG. 4 depicts a fleshy tissue site 10 for sensor attachment, such as a finger or thumb 400. The tissue 10 has an epidermis 12, a dermis 14, subcutaneous and other soft tissue 16 and bone 18.

FIG. 5 depicts a conventional pulse oximetry sensor 20 having a detector 22, an emitter 24 and a tape 26 attached to the fleshy tissue 10. Transmitted light 30 propagating from the emitter 24 to the detector 22 that results in a significant contribution to pulse oximetry measurements passes through and is absorbed by the pulsatile blood in the dermis 14. A portion of the transmitted light 30 is scattered out of the epidermis 12 and reflected by the tape 26 back into the fleshy tissue 10. The detector field-of-view (FOV) 40 is relatively wide and, as a result, the detector responds to transmitted light 30 that has propagated, at least in part, outside of the fleshy tissue 10.

FIG. 6 depicts a cyanotic infant sensor 300 that is configured to limit variations in the mean pathlength ratio. In particular, the sensor 300 has a light absorbing tape inner surface 324 that reduces transmitted light reflection back into the tissue site 10, as described with respect to FIGS. 3A-B, above. Further, the detector 342 has a limited FOV 50 so as to reduce the detection of transmitted light that has propagated outside of the tissue site 10, as described in detail with respect to FIGS. 7-9, below.

FIGS. 8-9 illustrate cyanotic infant sensor embodiments having a limited detector field-of-view (FOV). FIGS. 7A-B illustrate a conventional sensor 700 having a tape portion 760, a detector window 762 and a detector 742 having a relatively wide FOV 701. In particular, the window thickness does little to restrict the FOV. FIGS. 8A-B illustrate one embodiment of a cyanotic infant sensor 300 having a material portion 360, a detector window 362 and a detector 342 having a restricted FOV 801. In particular, the material thickness 360 functions to define the FOV 801. In one embodiment, the material thickness 360 comprises a flex circuit thickness and a base head tape thickness, as described with respect to FIG. 3B, above. FIGS. 9A-B illustrate another embodiment of a cyanotic infant sensor 900 having a material portion 960, a detector window 962 and a detector 942 having a restricted FOV 901. In particular, an O-ring 980 deposed around the window 962 defines the FOV 901.

FIG. 10 depicts an exemplar calibration surface 1000 for a cyanotic infant sensor 300 (FIGS. 3A-B) calculated along a DC response ratio axis 1001, a ratio-of-ratios axis 1003 and a resulting oxygen saturation axis 1005. Matching the emitters and detectors, as described with respect to FIG 3A, above, allows for pathlength calibration. In particular, variations in the detector DC response ratio ($RD_{dc}/IR_{dc}$) are attributed to variations in the mean pathlength ratio (EQ. 3). As such, a calibration surface is determined by statistical regression of experimental measurements obtained from human volunteers and calibrated measurements of oxygen saturation, as is done for a conventional calibration curve (FIG. 2). A calculated DC response ratio 1001 in combination with a conventionally calculated ratio-of-ratios 1003 is then used to derive an oxygen saturation 1005 for the calibration surface 1000.

A cyanotic infant sensor has been disclosed in detail in connection with various embodiments. These embodiments are disclosed by way of examples only and are not to limit the scope of the claims that follow. One of ordinary skill in art will appreciate many variations and modifications.

**Claims**

1. A pulse oximetry sensor (300) comprising:

a plurality of emitters (344) configured to transmit light having a plurality of wavelengths into a fleshy medium; the transmitted light being red and infrared light;

at least one detector (342) responsive to said light after absorption by constituents of pulsatile blood flowing within said medium so as to generate a plurality of intensity signals;

a flexible sensor head (320) housing said emitters and said at least one detector and comprising a flexible tape material (361) configured to position the emitters and detector on said fleshy medium by substantially conforming to a shape of said fleshy medium, said head having a light absorbing surface (324) adapted to be disposed proximate said medium, said light absorbing surface configured to reduce reflection of the transmitted light off of the flexible tape back into the fleshy medium;

a detector window (362) defined by said sensor head flexible tape material and by said light absorbing surface, so as to limit the field-of-view of said at least one detector.

2. The pulse oximetry sensor according to claim 1 wherein said sensor head is configured to attach to a human digit with said light absorbing surface disposed around the periphery of the digit so as to at least partially absorb emitter transmitted light that is scattered out of said digit.

3. The pulse oximetry sensor according to claim 2 wherein a material thickness of said sensor head proximate said detector window limits the field-of-view of said at least one detector.

4. The pulse oximetry sensor according to claim 2 further comprising an O-ring (962) disposed around the periphery of said detector window so as to limit the field-of-view of said at least one detector.

**Patentansprüche**

1. Pulsoxymetrie-Sensor (300) mit:

mehreren Emittern (344), die konfiguriert sind zum Ausstrahlen von Licht, das mehrere Wellenlängen aufweist, in ein fleischiges Medium; wobei das ausgestrahlte Licht rotes und infrarotes Licht ist;

mindestens einem Detektor (342), der auf das Licht nach Absorption durch Bestandteile von in dem Medium strömendem pulsierendem Blut anspricht, um mehrere Intensitätssignale zu erzeugen;

einem flexiblen Sensorkopf (320), in welchem die Emitter und der mindestens eine Detektor untergebracht sind, wobei der Sensorkopf ein flexibles Bandmaterial (361) aufweist, der konfiguriert ist, um die Emitter und den Detektor an dem fleischigen Medium zu positionieren, indem sie sich der Gestalt des fleischigen Mediums im Wesentlichen anpassen, wobei der Kopf eine lichtabsorbierende Oberfläche (324) aufweist, die geeignet ist, benachbart zu dem Medium angeordnet zu werden, wobei die lichtabsorbierende Oberfläche konfiguriert ist, Reflexion des ausgestrahlten Lichts von dem flexiblen Band zurück in das fleischige Medium zu reduzieren; und einem durch das flexible Bandmaterial des Sensorkopfs und die lichtabsorbierende Oberfläche definierten Detektorfenster (362) zur Begrenzung des Blickfelds des mindestens einen Detektors.

2. Pulsoxymetrie-Sensor nach Anspruch 1, wobei der Sensorkopf konfiguriert ist, an einem menschlichen Finger angebracht zu werden, wobei die lichtabsorbierende Oberfläche um den Umfang des Fingers herum angeordnet ist, um so vom Emitter ausgestrahltes Licht, das von dem Finger weg gestreut wird, zumindest teilweise zu absorbieren.

3. Pulsoxymetrie-Sensor nach Anspruch 2, wobei eine Materialdicke des Sensorkopfs in der Nähe des Detektorfensters das Blickfeld des mindestens einen Detektors begrenzt.

4. Pulsoxymetrie-Sensor nach Anspruch 2, ferner mit einem um den Rand des Detektorfensters herum angeordneten O-Ring (962) zur Begrenzung des Blickfelds des mindestens einen Detektors.

**Revendications**

1. Capteur d'oxymétrie de pouls (300) comprenant :

une pluralité d'émetteurs (344) configurés pour émettre une lumière ayant une pluralité de longueurs d'onde dans un milieu charnu ; la lumière émise étant une lumière rouge et infrarouge ;

au moins un détecteur (342) réagissant à ladite lumière après son absorption par les constituants du sang pulsé circulant dans ledit milieu de manière à générer une pluralité dé signaux d'intensité ;
une tête de capteur flexible (320) logeant lesdits émetteurs et ledit au moins un détecteur et comprenant un matériau en bande flexible (361) configuré pour positionner les émetteurs et le détecteur sur ledit milieu charnu en se conformant sensiblement à une forme dudit milieu charnu, ladite tête comportant une surface absorbant la lumière (324) conçue pour être disposée à proximité dudit milieu, ladite surface absorbant la lumière étant configurée pour réduire la réflexion de la lumière émise par la bande flexible de retour dans le milieu charnu ; une fenêtre de détecteur (362) définie par ledit matériau en bande flexible de tête de capteur et par ladite surface absorbant la lumière, de manière à limiter le champ de vision dudit au moins un détecteur.

2. Capteur d'oxymétrie de pouls selon la revendication 1, dans lequel ladite tête de capteur est configurée pour être attachée à un doigt humain avec ladite surface absorbant la lumière disposée autour de la périphérie du doigt de manière à absorber au moins partiellement la lumière émise par l'émetteur qui est diffusée hors dudit doigt.

3. Capteur d'oxymétrie de pouls selon la revendication 2, dans lequel une épaisseur de matériau de ladite tête de capteur à proximité de ladite fenêtre de détecteur limite le champ de vision dudit au moins un détecteur.

4. Capteur d'oxymétrie de pouls selon la revendication 2, comprenant en outre un joint torique (962) disposé autour de la périphérie de ladite fenêtre de détecteur de manière à limiter le champ de vision dudit au moins un détecteur.

FIG. 1 (Prior Art)

EP 1 771 109 B1

FIG. 2 (Prior Art)

FIG. 3A

FIG. 3B

EP 1 771 109 B1

FIG. 4          FIG. 5 (Prior Art)          FIG. 6

EP 1 771 109 B1

FIG. 9A

FIG. 9B
SECTION A-A

FIG. 8A

FIG. 8B
SECTION A-A

FIG. 7A (Prior Art)

FIG. 7B (Prior Art)
SECTION A-A

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6088607 A
- US 5482036 A
- US 6256523 B
- WO 9703603 A
- US 5437275 A